# EUROPEAN PATENT APPLICATION

(11) **EP 4 534 641 A1**
(43) Date of publication of application: **09.04.2025**
(21) Application number: 23811455.7
(22) Date of filing: 30.03.2023
(51) Int. Cl.: C12M 1/00, C12M 1/02, C12N 1/12, C12N 1/20, C12N 5/04

(54) **DEVICE FOR CULTURING PHOTOSYNTHETIC ORGANISM AND METHOD FOR CULTURING PHOTOSYNTHETIC ORGANISM USING SAME**

(30) Priority: 27.05.2022 JP 2022087214; 09.09.2022 JP 2022144212
(71) Applicant: Kansai Chemical Engineering Co., Ltd., Amagasaki-shi, Hyogo 660-0053 (JP); Bio-energy Corporation, Amagasaki-shi, Hyogo 660-0053 (JP)
(72) Inventor: NODA, Hideo, Amagasaki-shi, Hyogo 660-0053 (JP); HAMA, Shinji, Amagasaki-shi, Hyogo 660-0053 (JP); MUKAIDA, Tadahiro, Amagasaki-shi, Hyogo 660-0053 (JP)
(74) Representative: Schlich
(86) International application number: PCT/JP2023/013443
(87) International publication number: WO 2023/228576

(57) **Abstract**

A device for culturing a photosynthetic organism of the present invention includes: a culture tank for accommodating a culture medium containing a photosynthetic organism; and a liquid dispersion portion provided inside the culture tank. Here, the liquid dispersion portion includes one rotary shaft disposed along a vertical direction and at least one liquid flow member attached to the rotary shaft, the liquid flow member includes an ejection portion positioned above a liquid surface of the culture medium, a suction portion positioned below the liquid surface of the culture medium, and a flow path extending between the ejection portion and the suction portion and allowing the culture medium to flow therethrough, and at least a part of the culture tank includes a light-transmitting portion that allows transmission of light from the outside into the inside of the culture tank.

## Description

### Technical Field

The present invention relates to a device for culturing a photosynthetic organism, and a method for culturing a photosynthetic organism using the same.

### Background Art

Microalgae such as *Spirulina* and *Chlorella* are rich in nutrients that are important to humans and have been gaining attention as so-called "superfoods". These microalgae have also been reported to have beneficial effects, such as antioxidant effects, blood pressure regulation effects, cholesterol and blood sugar control, and anti-allergic effects, and are important as commercial materials that stimulate the purchasing intention of health-conscious consumers.

Further, in recent years, several reports have been published on techniques for large-scale culturing of microalgae (e.g., Non-Patent Documents 1 and 2), and it is anticipated that oils and fats produced by microalgae will be used as, for example, biofuels. It is expected that demand for microalgae will continue to increase in the future.

Meanwhile, techniques for improving the efficiency of industrial production of *Chlorella* and *Spirulina* have gaining a lot of attention. In particular, when culturing these algae, their photosynthetic function needs to be enhanced. Thus, overseas, outdoor culture facilities are built to engage in mass production in some cases. In such culture facilities, a huge culture pool is installed outdoors, and various types of algae contained in a culture medium are directly or indirectly irradiated with solar energy in the pool.

However, such a huge culture pool has some unique issues.

For example, it is difficult to irradiate the entire algae in the culture pool with solar energy more uniformly. Because there is a difference in solar energy received by algae to be cultured between a case where they are present near the liquid surface of the culture pool and a case where they are present at the bottom, the entire culture pool needs to be constantly stirred at an appropriate speed.

Next, in order to install a culture pool, a site needs to be flat, wide, and satisfy appropriate climatic conditions to obtain sufficient solar energy. In other words, it is difficult to install the above culture facilities on sloping land, in narrow sites such as around urban areas, cold areas, or in other areas where the weather is likely to change.

### Related Art Documents

### Non-Patent Documents

Non-Patent Document 1: Proceedings of Conference on Biomass Science 13(0),117-118, 2018 (General Incorporated Association The Japan Institute of Energy)
Non-Patent Document 2: Hitoshi Takeya and two others, "Development of stable mass culture technology for microalgae", [online], December 25, 2018, Kochi Deep Seawater Laboratory, [searched on May 23, 2022], Internet <URL: https://www.jpo.go.jp/system/laws/rule/guideline/patent/handbook_shinsa/document/in dex/01.pdf#page=28>

### Summary of Invention

### Problem to be Solved by the Invention

The present invention is to solve the above-descried problems, and it is an object thereof to provide a device for culturing a photosynthetic organism (also referred to as a "photosynthetic organism culture device") that can more efficiently culture photosynthetic organisms such as microalgae and is adaptable to a variety of production scales, and a method for culturing a photosynthetic organism using the device.

### Means for Solving the Problem

The present invention provides a device for culturing a photosynthetic organism, comprising:
a culture tank for accommodating a culture medium containing the photosynthetic organism; and
a liquid dispersion portion provided inside the culture tank,
wherein the liquid dispersion portion includes one rotary shaft disposed along a vertical direction and at least one liquid flow member attached to the rotary shaft,
the liquid flow member includes an ejection portion positioned above a liquid surface of the culture medium, a suction portion positioned below the liquid surface of the culture medium, and a flow path extending between the ejection portion and the suction portion and allowing the culture medium to flow therethrough, and
at least a part of the culture tank includes a light-transmitting portion that allows transmission of light from an outside into an inside of the culture tank.

In one embodiment, the light-transmitting portion is provided in at least one region selected from the group consisting of a wall surface portion and a lid portion of the culture tank.

In a further embodiment, the light-transmitting portion is provided on a wall surface portion of the culture tank, the culture medium is ejected from the ejection portion of the liquid flow member by rotating the rotary shaft in the liquid dispersion portion, and the culture medium flows down on an inner side along the wall surface portion of the culture tank to form a thin film.

In one embodiment, the liquid flow member is inclined such that the suction portion is closer to the rotary shaft than the ejection portion is.

In one embodiment, the liquid dispersion portion includes a plurality of the liquid flow members around an axis of the rotary shaft.

In one embodiment, the liquid flow member has a cylindrical shape with open ends.

In one embodiment, the liquid flow member has a gutter-like shape.

The present invention also provides a device for culturing a photosynthetic organism, comprising:
a culture tank for accommodating a culture medium containing a photosynthetic organism; and
a liquid dispersion portion provided inside the culture tank,
wherein the liquid dispersion portion includes one rotary shaft disposed along a vertical direction and at least one liquid flow member attached to the rotary shaft,
the liquid flow member includes an ejection portion positioned above a liquid surface of the culture medium, a suction portion positioned below the liquid surface of the culture medium, and a flow path extending between the ejection portion and the suction portion and allowing the culture medium to flow therethrough, and
the device includes an internal light source disposed inside the culture tank.

The present invention also provides a method for culturing a photosynthetic organism, comprising:
stirring a culture medium containing the photosynthetic organism under light irradiation, by rotating a rotary shaft in the above device for culturing a photosynthetic organism.

In one embodiment, the light is emitted toward the light-transmitting portion provided in the culture tank in the device for culturing a photosynthetic organism.

In one embodiment, wherein the photosynthetic organism is at least one organism selected from the group consisting of photosynthetic bacteria, blue-green algae, microalgae, and plant cells.

### Effects of the Invention

According to the present invention, the culture medium can be efficiently mixed up by moving the scooped up culture medium to a position above the liquid surface and dispersing it. At this time, for example, in addition to stirring based on horizontal rotation, vertical movement and circulation can be facilitated for the culture medium contained in the culture tank. Also, the culture medium mixed up in this manner can receive light through a light transmitting portion of the culture tank, and as a result, photosynthesis of the photosynthetic organism contained in the culture medium can be effectively facilitated.

### Brief Description of Drawings

FIG. 1 is a schematic view showing an example of a photosynthetic organism culture device of the present invention.
FIG. 2 is a perspective view schematically showing an example of a wall surface portion and a bottom that constitute a culture tank of the culture device shown in FIG. 1.
FIG. 3(a) is an end view of the culture device taken along the line A-Ain FIG. 1, and FIG. 3(b) is an end view of the culture device taken along the line B-B in FIG. 1.
FIG. 4 is a perspective view schematically showing an example of a liquid flow member constituting a liquid dispersion portion of the culture device shown in FIG. 1.
FIG. 5 is a schematic view showing another example of the photosynthetic organism culture device of the present invention.
FIG. 6(a) is an end view of the culture device taken along the line A'-A' in FIG. 5, and FIG. 6(b) is an end view of the culture device taken along the line B'-B'in FIG. 5.
FIG. 7 is a perspective view schematically showing an example of a liquid flow member constituting the liquid dispersion portion of the culture device shown in FIG. 5.
FIG. 8 is a schematic view showing yet another example of the photosynthetic organism culture device of the present invention.
FIG. 9 is a schematic view showing yet another example of the photosynthetic organism culture device of the present invention.
FIG. 10 is a longitudinal cross-sectional view schematically showing an example of a liquid flow member constituting the liquid dispersion portion of the culture device shown in FIG. 9.
FIG. 11 is a diagram schematically showing an example of an obstruction plate that can be incorporated into the photosynthetic organism culture device of the present invention, the obstruction plate being constituted by a combination of two sets of plates.
FIG. 12 is a schematic view showing yet another example of the photosynthetic organism culture device of the present invention.
FIG. 13 is a schematic view showing yet another example of the photosynthetic organism culture device of the present invention.
FIG. 14 is a schematic view of a photosynthetic organism culture device (R1) fabricated in Example 1, and FIG. 14(b) is a schematic view of a liquid dispersion portion used instead of that of the above device (R1) in order to fabricate a photosynthetic organism culture device (R2) of Comparative Example 1.
FIG. 15 is a graph showing the results of culturing microalgae (*Euglena*) using the photosynthetic organism culture device (R1) fabricated in Example 1 and the photosynthetic organism culture device (R2) fabricated in Comparative Example 1.
FIG. 16 is a graph showing the results of culturing microalgae *(Euglena)* in a state in which a mantle is provided in a portion of the photosynthetic organism culture device (R1) fabricated in Example 1 and a portion of the photosynthetic organism culture device (R2) fabricated in Comparative Example 1.
FIG. 17 is a graph showing the results of culturing microalgae (*Chlorella*) using the photosynthetic organism culture device (R1) fabricated in Example 1 and the photosynthetic organism culture device (R2) fabricated in Comparative Example 1.

### Description of Embodiments

### Photosynthetic Organism Culture Device

A device for culturing a photosynthetic organism (also referred to as a "photosynthetic organism culture device") of the present invention will be described with reference to the accompanying drawings. Note that constituent elements denoted by the same reference numerals in the following drawings are the same as those shown in the other drawings.

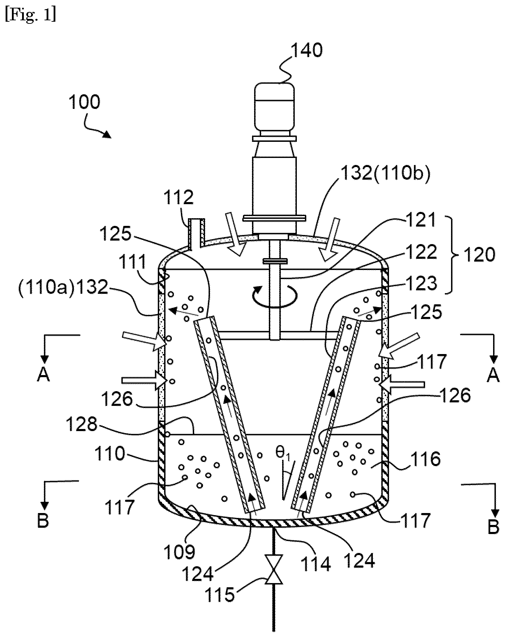

FIG. 1 is a schematic view showing an example of a photosynthetic organism culture device of the present invention. A photosynthetic organism culture device (may be simply referred to as a "culture device" hereinafter) 100 includes a culture tank 110 and a liquid dispersion portion 120.

The culture tank 110 is a sealable tank in which a culture medium 116 containing photosynthetic organisms 117 can be accommodated and stirred, and has a wall surface portion 110a having a shape such as a circular cylindrical, elliptical cylindrical, or rectangular cylindrical shape, and a bottom 109 that is a flat bottom, a round bottom, a conical bottom, or a downward sloping bottom, for example. The top of the culture tank 110 may have a lid portion 110b having an openable structure, such as a maintenance hole, for example. Alternatively, the top of the culture tank 110 may be open without being provided with a lid portion.

In the present invention, at least a part of the culture tank 110 includes light-transmitting portions 132 that allow transmission of light from an outside into the culture tank 110.

For example, as shown in FIG. 1, a wall surface portion 110a and/or a lid portion 110b of the culture tank 110 includes the light-transmitting portions 132. The area of the light-transmitting portions 132 is not particularly limited, for example, but is designed to be as large as possible within a range in which a sufficient strength is obtained, in order to increase the culture efficiency of the photosynthetic organisms 117 in the culture tank 110 described below. For example, the entire culture tank 110 may be constituted by the light-transmitting portion 132 that allows transmission of light from the outside.

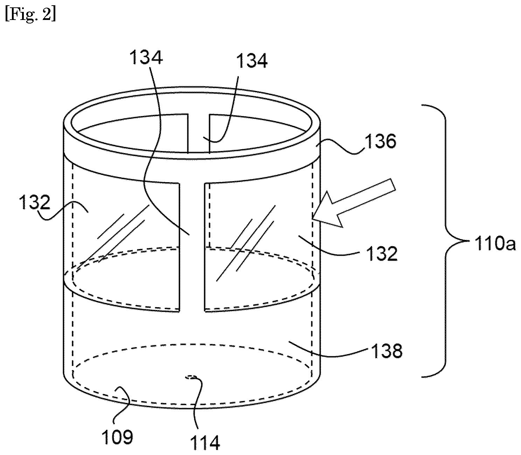

FIG. 2 is a perspective view schematically showing an example of the wall surface portion 110a and the bottom 109 that constitute the culture tank 110 of the culture device 100 shown in FIG. 1.

As shown in FIG. 2, for example, the wall surface portion 110a is constituted by the light-transmitting portions 132 and other portions (e.g., supports 134, an upper frame 136, and a lower frame 138), and is formed as a single body with the bottom 109.

The light-transmitting portions 132 are made of, for example, a material having a high light transmittance. Examples of materials that can form the light-transmitting portions 132 include glass (e.g., reinforced glass, quartz glass), transparent resin (e.g., acrylic resin, polycarbonate, polyethylene terephthalate, polyvinyl chloride, polystyrene, AS resin, transparent ABS resin, transparent polyurethane, silicone resin) and combinations thereof. The light-transmitting portions 132 may be colorless and transparent or colored and transparent. Afilm that partially blocks light wavelengths may be adhered to the outside thereof, as long as light from the outside suitable for culturing photosynthetic organisms can be efficiently transmitted into the inside. The outer and inner shapes of the light-transmitting portions 132 are designed, for example, such that they respectively match the outer shapes and the inner shapes of the upper frame 136 and the lower frame 138.

The supports 134 are provided to connect the upper frame 136 and the lower frame 128 and increase the longitudinal strength of the wall surface portion 110a. Although two supports 134 are provided in FIG. 2, there is no particular limitation on the number of supports 134. It is preferable that when a plurality of supports 134 are provided, the spacings between supports 134 are approximately equal to each other because light is allowed to enter the culture tank from the outside more evenly through the light-transmitting portions 132. Note that when the light-transmitting portions 132 have sufficient strength, the wall surface portion 110a may not be provided with the supports 134.

The upper frame 136 is also provided as needed to increase the strength of the entire wall surface portion 110a. When the culture tank 110 shown in FIG. 1 has the lid portion 110b, the upper frame 136 is provided to prevent the lid portion 110b from coming into direct contact with the light-transmitting portions 132 and damaging the light-transmitting portions 132.

The lower frame 138 supports the light-transmitting portions 132 from below, and can store the culture medium therein.

The supports 134, the upper frame 136, and the lower frame 138 are made of materials (e.g., a metal such as iron, stainless steel, hastelloy, or titanium, or combinations thereof) having a higher strength than the light-transmitting portions 132. These components may be given internal coatings known in the art, such as Teflon (registered trademark), glass lining, or rubber lining, to enhance chemical resistance.

Referring again to FIG. 1, the size (capacity) of the culture tank 110 is not necessarily limited, because it is set as appropriate according to the type of photosynthetic organisms 117 cultured in the culture device 100, the amount of culture medium, and the like, but the size is from 0.1 liters to 100,000 liters, for example. Note that the culture tank 110 may have a form such as a glass jar fermenter whose entirety is the light-transmitting portion 132, or a single-use transparent resin bag (a culture bag) that can be used in fields of biopharmaceutical production and the like. Alternatively, portions of the culture tank 110 other than the light-transmitting portions 132 may be made of one or a combination of materials such as iron, stainless steel, hastelloy, titanium, and concrete. For example, portions of the culture tank 110 other than the light-transmitting portions 132 may be constituted by a combination of a side portion made of the above-mentioned high-strength material such as stainless steel, hastelloy, or titanium and a bottom made of concrete. A structure in which the entire culture tank 110 serves as the light-transmitting portion 132 can be adopted when the culture tank 110 has a relatively small capacity (e.g., 1 litter to 200 liters).

In an embodiment, the culture tank 110 further includes a culture medium inlet 112 and a culture medium outlet 114. The culture medium inlet 112 is an inlet through which the culture medium 116 is to be newly supplied into the culture tank 110. The culture medium inlet 112 is provided on the top (e.g., on the lid portion 110b) of the culture tank 110, for example. Alternatively, the culture medium inlet 112 may be provided on the wall surface portion 110a of the culture tank 110. The number of culture medium inlets 112 provided on the culture tank 110 is not limited to one. For example, a plurality of culture medium inlets may be provided on the culture tank 110.

The culture medium outlet 114 is an outlet through which the photosynthetic organisms 117 cultured in the culture tank 110 or a product (e.g., oil and fat) thereof is to be taken out of the culture tank 110. The culture medium outlet 114 is capable of discharging effluent and the like in addition to the photosynthetic organisms 117 and the product, and the discharge can be regulated by opening and closing a valve 115 provided downstream of the culture medium outlet 114, for example. The culture medium outlet 114 is provided in connection with the center of the bottom 109 in the culture tank 110 in FIG. 1, for example, but is not particularly limited to this arrangement.

The liquid dispersion portion 120 is provided inside the culture tank 110, and is constituted by one rotary shaft 121 disposed along the vertical direction inside the culture tank 110, and liquid flow members 123 attached to the rotary shaft 121 via an attachment 122 extending preferably in the horizontal direction. The rotation of the rotary shaft 121 and centrifugal force caused by the rotation and applied to the liquid flow members 123 can cause the liquid dispersion portion 120 to scoop up the culture medium 116 contained in the culture tank 110 and cause the culture medium to flow upward from the lower side of the culture tank 110. The liquid flow members 123 each include a suction portion 124, an ejection portion 125, and a flow path 126 extending between the suction portion 124 and the ejection portion 125. The suction portion 124 is disposed below a liquid surface 128 of the culture medium 116, and the ejection portion 125 is disposed above the liquid surface 128 of the culture medium 116.

In the present invention, the arrangement of the suction portions 124 and the ejection portions 125 described above is preferably maintained not only in a static stage (i.e., a state in which the rotary shaft 121 is not rotated and the liquid surface of the culture medium 116 is spread substantially in the horizontal direction) but also in a stage in which the rotary shaft 121 is rotated at a desired rotational speed (i.e., a state in which the culture medium 116 is stirred as described later through the rotation of the rotary shaft 121). As a result, the culture medium 116 in the culture tank 110 can be easily scooped up from the suction portions 124 of the liquid flow members due to the rotation of the rotary shaft 121 and the liquid flow members 123, moved by centrifugal force through the flow paths 126 in the liquid flow members 123 to the ejection portions 125, and then ejected from the ejection portions 125 toward an inner wall 111 of the culture tank 110 or the liquid surface 128 of the culture medium 116, for example.

The rotary shaft 121 is a shaft with a predetermined rigidity, and has, for example, a circular cylindrical or round columnar shape. The rotary shaft 121 is typically disposed along the vertical direction inside the culture tank 110. The thickness of the rotary shaft 121 is not necessarily limited, but is 8 mm to 200 mm, for example. The length of the rotary shaft 121 varies in accordance with the size of the culture tank 110 used or the like, and an appropriate length may be selected by a person skilled in the art.

In the culture device 100 of the present invention, an end of the rotary shaft 121 is connected to a motor 140 or other rotating means at a position above the culture tank 110. The other end of the rotary shaft 121 is not connected to the bottom 109 of the culture tank 110, and is disposed at a certain distance from the bottom 109 of the culture tank 110 (preferably disposed at a distance from the liquid surface 128 of the culture medium 116), for example. This reduces the risk of the rotary shaft 121 coming into contact with the culture medium 116. Alternatively, the other end of the rotary shaft may be housed in a bearing provided at the bottom 109 of the culture tank.

Note that an optical sensor (not shown) may be electrically connected to the motor 140 in the culture device 100 of the present invention. For example, when the culture device 100 is installed outdoors and the optical sensor detects sunlight, a predetermined signal is sent to the motor 140, causing the motor 140 to start rotating. When it becomes night and the optical sensor can no longer detect sunlight, a signal indicating that the optical sensor can no longer detect sunlight is sent to the motor 140, causing the motor 140 to stop rotating.

Further, the motor 140 may be electrically connected to a solar unit (not shown). For example, when the culture device 100 is installed outdoors and the solar unit is irradiated with sunlight, a predetermined current flows through the motor 140, causing the motor 140 to rotate. When it becomes night and the solar unit can no longer detect sunlight, the current from the solar unit to the motor 140 is cut off, causing the motor 140 to stop rotating.

By electrically connecting the solar unit to a storage battery to facilitate charging of the battery during the day, light from a light-emitting body such as an LED light can also be irradiated onto the culture device 100 using the storage battery at night.

In the culture device 100 shown in FIG. 1, the liquid flow members 123 are each inclined such that the suction portion 124 is closer to the rotary shaft 121 than the ejection portion 125 is. In FIG. 1, the liquid flow members 123 are each attached so as to be inclined to form a predetermined angle (alternatively referred to as an "attachment inclination angle") θ₁ with respect to the axial direction of the rotary shaft 121. The attachment inclination angle θ₁ may be set to any angle by a person skilled in the art, but is, for example, 10° to 60° and preferably 15° to 45°.

In the culture device 100 shown in FIG. 1, as the liquid dispersion portion 120, two liquid flow members 123 are provided symmetrically around the rotary shaft 121. The number of liquid flow members that can be provided in the culture device of the present invention is not necessarily limited, but is, for example, one or more, preferably from 2 to 8, and more preferably from 2 to 6. These liquid flow members are preferably attached at substantially equal angle intervals around the axis of the rotary shaft.

In the present invention, the liquid flow members 123 may be processed to be entirely cylindrical (e.g., circular cylindrical, elliptic cylindrical, or angular cylindrical), may have a so-called gutter-like shape such as a semi-circular cylindrical, semi-angular cylindrical, or V letter shape, may have such a gutter-like shape at the lower end and the upper end with an intermediate portion therebetween being processed to be cylindrical (e.g., circular cylindrical, elliptic cylindrical, or angular cylindrical), or may have such a gutter-like shape only at the lower end or only at the upper end with the other portion being processed to be cylindrical (e.g., circular cylindrical, elliptic cylindrical, or angular cylindrical), for example.

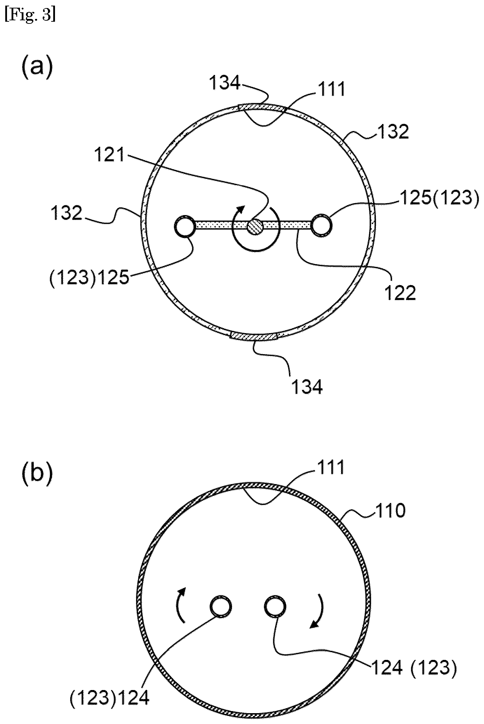

For example, in the embodiment shown in FIG. 1, the liquid flow members 123 have a cylindrical shape with open ends. If such cylindrical liquid flow members 123 are employed, two liquid flow members 123 are attached around the axis of the rotary shaft 121 by means of the attachment 122 at a substantially equal distance from the rotary shaft 121 in a horizontal cross section in the upper portion of the culture tank 110 in the culture device 100 (e.g., near the ejection portions 125 of the liquid flow members 123 taken along the line A-A in FIG. 1) as shown in FIG. 3(a), for example. When the rotary shaft 121 rotates, the two liquid flow members 123 can be rotated via the attachment 122 along a portion closer to the inner wall 111 than to the center of the culture tank 110. Meanwhile, the two liquid flow members 123 are positioned close to the center of the culture tank 110 in a horizontal cross section in the lower portion of the culture tank 110 in the culture device 100 (e.g., near the suction portions 124 of the liquid flow members 123 taken along the line B-B in FIG. 1) as shown in FIG. 3(b), for example. When the rotary shaft 121 rotates, the two liquid flow members 123 can be rotated along a portion close to the center of the culture tank 110.

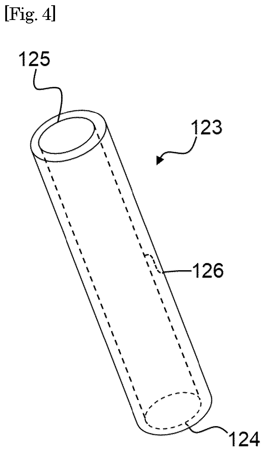

The size of the liquid flow members 123 is not particularly limited, but, for example, if a circular cylindrical member as shown in FIG. 4 is employed, the inner diameter of the circular cylindrical portion is 2 mm to 200 mm, for example. The length from the suction portion 124 to the ejection portion 125 (i.e., the length of the path 126) is 40 mm to 8,000 mm, for example.

Referring again to FIG. 1, the culture medium 116 contained in the culture tank 110 contains the photosynthetic organisms 117 and water, and other components.

The photosynthetic organisms 117 are organisms that can utilize light to facilitate photosynthesis and grow and/or produce products such as oils and fats, and examples of the photosynthetic organisms 117 include photosynthetic bacteria, blue-green algae, microalgae, and plant cells, and combinations thereof.

Photosynthetic bacteria are photosynthetic prokaryotes other than cyanobacteria, and examples of photosynthetic bacteria include purple bacteria, green sulfur bacteria, green filamentous (filamentous) bacteria, and heliobacteria. These bacteria have the ability to perform anoxygenic photosynthesis, which does not produce oxygen.

Blue-green algae, which are also known as cyanobacteria, are eubacteria that perform oxygenic photosynthesis.

There are a wide variety of microalgae. For example, the green alga *Chlamydomonas reinhardtii* (Japanese name: Konamidorimushi) is a eukaryotic photosynthetic organism with the simplest cell structure, and is used as a model organism that bridges a gap between higher plants (*Arabidopsis thaliana*) and cyanobacteria. Examples of microalgae include unicellular microalgae (e.g., green algae such as *Chlorella, Chlamydomonas, Scenedesmus,* and *Dunaliella*; *Euglena;* red algae such as the genus *Porphyridium,* the genus *Rhodella,* the genus *Cyanidiumm,* and the genus *Galdieria*; golden algae such as the genus *Ochromonas*; diatoms such as the genus *Chaetocerus* and the genus *Nitschia*), as well as multicellular microalgae (e.g., ulvophytes such as the genus *Bryopsis,* the genus *Codium,* the genus *Ulva,* and the genus *Acetabularia;* brown algae such as the genus *Sarugassum,* the genus *Ecklonia,* and the genus *Scytosiphon;* and red algae such as the genus *Porphyra,* the genus *Aglaothamnion,* and the genus *Anthithamnion*).

Plant cells are, for example, cultured cells formed from a part of a plant body, and examples thereof include cultured plant cells collected from a part (e.g., leaves or roots) of a plant such as a tobacco plant, arabidopsis, a rice plant, a poplar, or Japanese yew. The cells have properties such as the ability to grow more uniformly and quickly in suspension than ordinary plant bodies and to synthesize substances useful as secondary metabolites. The obtained secondary metabolites can be used not only in scientific research but also in the development of gene expression regulation and culture techniques for producing pharmaceutical intermediates and the like.

Other components that may be contained in the culture medium 116 are not necessarily limited, but are, for example, carbon sources, nitrogen sources, vitamins, amino acids, and inorganic salts, as well as combinations thereof, which are required for growth and proliferation of the photosynthetic organisms. The content of the other components that may be contained in the culture medium 116 is not particularly limited, and an appropriate amount or concentration can be selected as appropriate by one skilled in the art.

According to the culture device 100 of the present invention, the rotation of the rotary shaft 121 causes the liquid flow members 123 of the liquid dispersion portion 120 to scoop up the culture medium 116 from the suction portions 124. The scooped up culture medium is moved through the paths 126 to the ejection portions 125 by centrifugal force caused by the rotation of the rotary shaft 121, and then ejected from the ejection portions 125 into the culture tank 110, specifically, to portions above the liquid surface 128 of the culture medium 116 in the culture tank 110. This allows the culture medium 116 to collide with the inner wall 111 of the culture tank 110 and the liquid surface 128 and to move upward from the bottom 109 of the culture tank 110, thereby facilitating mixing up (e.g., stirring or circulation in the vertical direction) of the culture medium 116 in the height direction of the culture tank 110.

On the other hand, the inside of the culture tank 110 is irradiated with light from the outside through the light-transmitting portions 132 provided in the culture tank 110. Inside the culture tank 110, for example, in addition to the photosynthetic organisms 117 stored in the culture tank 110, the photosynthetic organisms 117 contained in the culture medium 116 ejected from the liquid flow members 123 of the liquid dispersion portion 120 are irradiated with light. In particular, the photosynthetic organisms 117 ejected from the ejection portions 125 of the liquid flow members 123 and flowing down along the inner wall 111 (i.e., the inner wall surfaces of the light-transmitting portions 132) of the culture tank 110 in the state of the culture medium can directly receive light from the light-transmitting portions 132. As the culture medium 116 is mixed up, the photosynthetic organisms 117 can flow down along the inner surface walls of the light-transmitting portions almost continuously at all times, which further facilitates photosynthesis of the photosynthetic organisms 117.

As a result, the photosynthetic organisms 117 in the culture device 100 can be effectively cultured.

Note that the rotary shaft 121, the attachment 122, and the liquid flow members 123 are each independently made of materials such as iron, stainless steel, hastelloy, titanium, or other metals, or combinations thereof, for example. These components may also be given coatings known in the art, such as Teflon (registered trademark), glass lining, or rubber lining, to enhance chemical resistance.

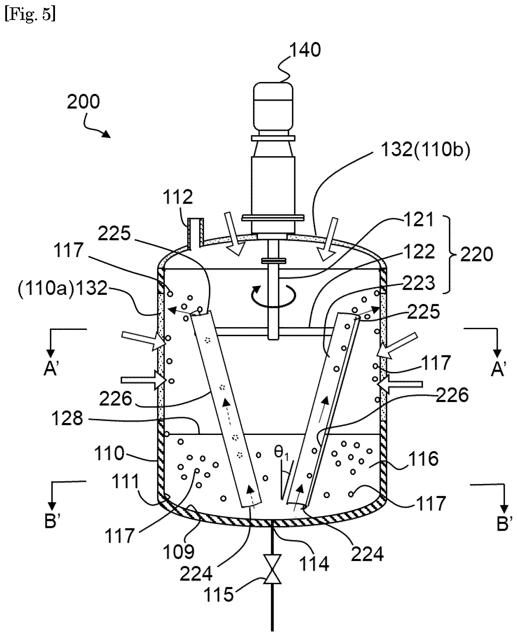

FIG. 5 is a schematic view showing still another example of the culture device of the present invention. In a culture device 200, flow paths 226 of liquid flow members 223 constituting a liquid dispersion portion 220 each have a gutter-like shape.

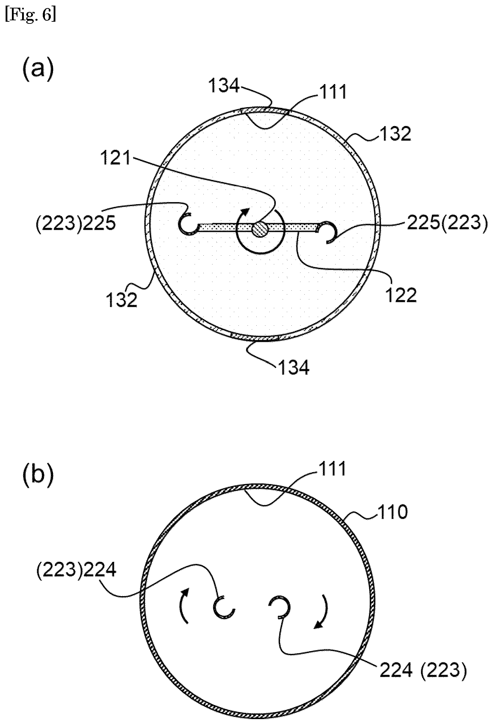

If such gutter-like liquid flow members 223 are employed, two liquid flow members 223 are attached around the axis of the rotary shaft 121 by means of the attachment 122 at a substantially equal distance from the rotary shaft 121 such that opening portions of the liquid flow members 223 are oriented in the moving direction (tangential direction) of the circular motion in a horizontal cross section in the upper portion of the culture tank 110 in the culture device 200 (e.g., near ejection portions 225 of the liquid flow members 223 taken along the line A-A in FIG. 5) as shown in FIG. 6(a), for example. When the rotary shaft 121 rotates, the two liquid flow members 223 can be rotated via the attachment 122 along a portion closer to the inner wall 111 than to the center of the culture tank 110. Meanwhile, the two liquid flow members 223 are positioned close to the center of the culture tank 110 in a horizontal cross section in the lower portion of the culture tank 110 in the culture device 200 (e.g., near the suction portions 224 of the liquid flow members 223 taken along the line B'-B' in FIG. 5) as shown in FIG. 6(b), for example. When the rotary shaft 121 rotates, the two liquid flow members 223 can be rotated along a portion close to the center of the culture tank 110.

The rotation of the rotary shaft 121 can cause the gutter-like liquid flow members 223 as used in the culture device 200 in FIG. 5 to scoop up not only the culture medium 116 near the suction portions 224 but also the culture medium 116 positioned at portions in which the liquid members 223 are immersed above the suction portions 224. The scooped up culture medium 116 is then moved through the flow paths 226 of the liquid flow members 223 and then ejected from the ejection portions 225, and thus stirring or circulation of the culture medium 116 (and the photosynthetic organisms 117 contained therein) in the height direction of the culture tank 110 can be facilitated. The centrifugal force caused by the rotation of the rotary shaft 121 and applied to the lower portions of the liquid flow members 223 is relatively small and the density of the culture medium 116 that is scooped up is large, and thus the amount of liquid moving up through the flow paths 226 of the liquid flow members 223 may not be very much. Therefore, when such gutter-like liquid flow members 223 are used, the liquid flow members 223 can operate as conventional stirring blades as well.

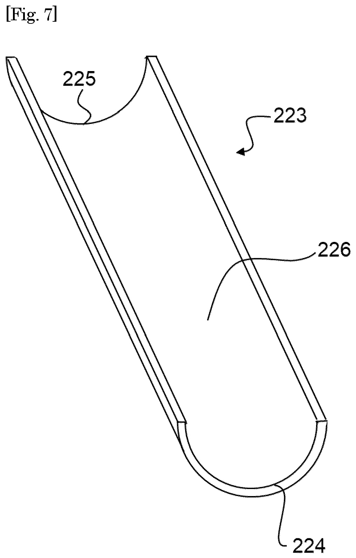

The size of the gutter-like liquid flow members 223 is not particularly limited either, but, for example, if a liquid flow member having a semi-circular cylindrical flow path 226 as shown in FIG. 7 is employed, the diameter of the semi-circular cylindrical portion is 2 mm to 200 mm, for example. The length from the suction portion 224 to the ejection portion 225 is 40 mm to 8,000 mm, for example. The materials that can form the gutter-like liquid flow members 223 are the same as those that form the cylindrical liquid flow members 123 shown in FIG. 1 above and the like.

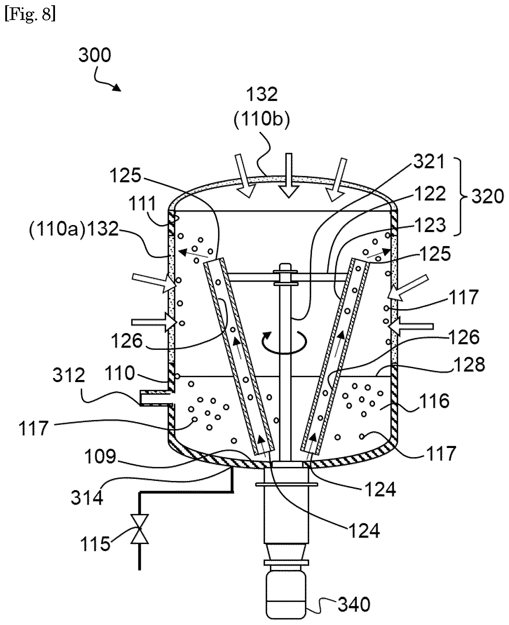

FIG. 8 is a schematic view showing still another example of the culture device of the present invention. In a culture device 300 shown in FIG. 8, a motor 340 is provided in the lower portion of a culture tank 110 (i.e., a bottom 109 of the culture tank 110). A rotary shaft 321 that constitutes a liquid dispersion portion 320 is attached to an upper portion of the motor 340, extends in the vertical direction, and is connected to the cylindrical liquid flow members 123 via the attachment 122. In FIG. 8, the culture medium inlet 312 is provided in a wall surface portion 110a of the culture tank 110, and a culture medium outlet 314 is disposed at any position on the bottom 109 that does not interfere with the motor 340.

With such an arrangement, the light-transmitting portions 132 can be provided on the entire surface of the top (in particular, a lid portion 110b) of the culture tank 110, for example. As a result, more light from the outside enters the inside of the culture tank 110 through the light-transmitting portions 132 of the lid portion 110b, and thus photosynthesis of the photosynthetic organisms 117 in the culture tank 110 can be further facilitated.

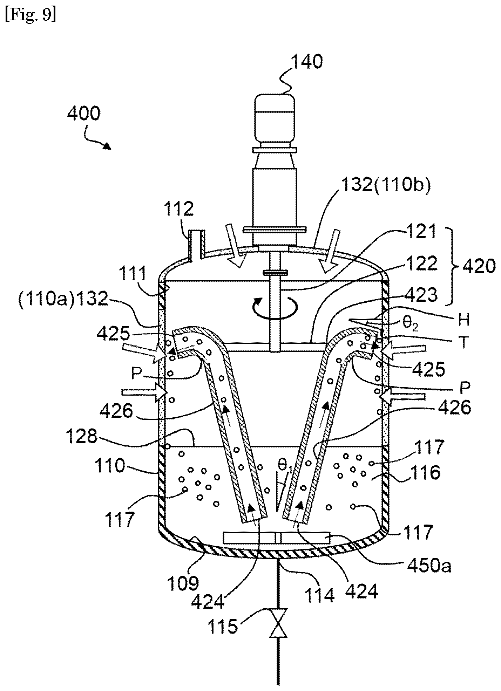

FIG. 9 is a schematic view showing yet another example of the photosynthetic organism culture device of the present invention. In a culture device 400, liquid flow members 423 constituting the liquid dispersion portion 420 each include a suction portion 424, an ejection portion 425, and a cylindrical flow path 426 extending between the suction portion 424 and the ejection portion 425.

As shown in FIG. 9, in the liquid dispersion portion 420, the suction portions 424 of the liquid flow members 423 are disposed below a liquid surface 128 of the culture medium 116, the ejection portions 425 of the liquid flow members 423 are disposed above the liquid surface 128 of the culture medium 116. The rotation of the rotary shaft 121 and centrifugal force caused by the rotation and applied to the liquid flow members 423 can cause the liquid dispersion portion 420 to scoop up, from the suction portions 424 of the liquid flow members 423, the culture medium 116 contained in the culture tank 110 as shown in FIG. 9 and cause the culture medium 116 to flow upward from the lower side of the culture tank 110 through the cylindrical flow paths 426 together with the photosynthetic organisms 117. Subsequently, the scooped up culture medium is ejected from the ejection portions 425 of the liquid flow members 423 together with the photosynthetic organisms 117 to a position above the liquid surface 128.

In general, according to the siphon principle, when a tube is filled with liquid and liquid is ejected from an outlet (corresponding to the ejection portion in the present invention) provided at a low position, new liquid is sucked up from an inlet (corresponding to the suction portion in the present invention) provided at a high position, and the liquid continuously moves from the inlet to the outlet until all the liquid present at the inlet has been moved away or until air bubbles enter the tube and begin to cause cavitation.

In contrast, in the present invention, when the culture medium moves from the suction portions 424 to the ejection portions 425 through the cylindrical flow paths 426 in the liquid flow members 423, once the cylindrical flow paths 426 are filled with the culture medium, the liquid dispersion members 423 continuously allow movement of the culture medium from suction of the culture medium at the suction portions 424 through the cylindrical flow paths 426 to ejection of the culture medium at the ejection portions 425, just like a tube used in the siphon principle, while rotation of the rotary shaft 121 continues. Such continuous movement of a culture medium is referred to as "siphon-like" movement of the culture medium in this specification. In the present invention, once the siphon-like movement of the culture medium occurs, the amount of culture medium moving per unit time (e.g., mL/sec) increases compared to the amount of culture medium (e.g., mL/sec) before the continuous movement occurs in the case of the same rotation of the rotary shaft 121, thereby enabling a large amount of culture medium to be mixed up efficiently.

In the liquid dispersion portion 420, it is possible to create the above-mentioned siphon-like movement of the culture medium among the suction portions 424, the cylindrical flow paths 426, and the ejection portions 425 that constitute the liquid flow members 423. As a result, once the siphon-like movement of the culture medium is started, even when the rotation of the rotary shaft 121 is comparatively suppressed (i.e., the power required for rotation is reduced), suction of the culture medium (including the photosynthetic organisms) from the suction portions 424 and ejection of the culture medium (including the photosynthetic organisms) from the ejection portions 425 can be substantially continuously carried out in the liquid flow members 423.

In the present invention, the arrangement of the suction portions 424 and the ejection portions 425 with respect to the liquid surface 128 is preferably maintained not only in a static stage (i.e., a state in which the rotary shaft 121 is not rotated and the liquid surface of the culture medium 116 is spread substantially in the horizontal direction) but also in a stage in which the rotary shaft 121 is rotated at a desired rotational speed (i.e., a state in which the culture medium 116 is stirred through the rotation of the rotary shaft 121). As a result, the culture medium 116 in the culture tank 110 can be easily scooped up, together with the photosynthetic organisms 117, from the suction portions 424 of the liquid flow members due to the rotation of the rotary shaft 121 and the liquid flow members 423, moved by centrifugal force through the cylindrical flow paths 426 in the liquid flow members 423 to the ejection portions 425, and then ejected from the ejection portions 425 toward an inner wall 111 of the culture tank 110 or the liquid surface 128 of the culture medium 116, for example.

In the liquid dispersion portion 420, at least parts of the cylindrical flow paths 426 are bent (as indicated by bent portions P in FIG. 9), and the ejection portions 425 are oriented downward. As a result, the culture medium scooped up from the suction portions 424 of the liquid dispersion portion 420 is usually ejected from the ejection portions 425 of the liquid dispersion portion 420 in the culture tank 110 together with the photosynthetic organisms toward a position below the height of the ejection ports 425.

Although one cylindrical flow path 426 is provided with one bent portion P in the liquid dispersion portion 420 shown in FIG. 9, the present invention is not limited to such a form as long as the ejection portion 425 is oriented downward (e.g., downward relative to the horizontal direction). One cylindrical flow path 426 may be provided with a plurality of bent portions as long as the ejection portion 425 is oriented downward. In the present invention, it is preferable that the bent portions P are curved in an arc shape in order to avoid or reduce undesired erosion at the bent portions P.

The liquid flow members 423 are processed to be entirely cylindrical (e.g., circular cylindrical, elliptic cylindrical, or angular cylindrical), for example.

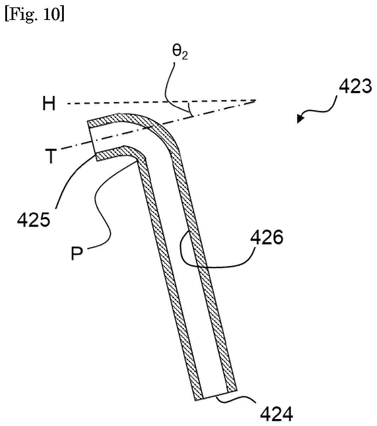

The size of the liquid flow members 423 is not particularly limited. FIG. 10 is a longitudinal cross-sectional view schematically showing an example of a liquid flow member constituting the liquid dispersion portion of the culture device shown in FIG. 9. For example, if a cylindrical member is used in the liquid dispersion member 423, the inner diameter of the circular cylindrical portion is 2 mm to 200 mm, for example. The length from the suction portion 424 to the bent portion P is not particularly limited, and an appropriate length may be selected by a person skilled in the art. In FIG. 10, the inner diameter of the suction portion 424, the inner diameter of the cylindrical path 426, and the inner diameter of the ejection portion 425 are shown as if they were of approximately the same size, but the present invention is not limited to this. For example, the inner diameter of the liquid flow member 423 may gradually or stepwise decrease from the suction portion 424 to the ejection portion 425 through the bent portion P.

In the liquid flow member 423 shown in FIG. 10, an angle θ₂ between a direction T in which the ejection portion 425 is oriented and a horizontal direction H is, for example, -90°≤θ₂≤10°, or as another example, -60°≤θ₂≤0°, with respect to the horizontal direction. If the angle θ₂ is below -90°, the culture medium may flow backward in the liquid flow member, making it impossible for the culture medium to be effectively ejected from the ejection portion together with the photosynthetic organisms. If the angle θ₂ exceeds 10°, even when the inside of the cylindrical path 120 is filled with the culture medium through rotation, the culture medium does not move in a siphon-like manner, and as a result, it may become difficult to reduce power applied to the culture device.

On the other hand, the obstruction plate 450a is positioned at substantially the center of the bottom 109 of the culture tank 110 in the culture device 400 shown in FIG. 9. Apredetermined gap is provided between the obstruction plate 450a and the suction portion 424 of each liquid flow member 423. Even when the liquid dispersion portion 420 is rotated around the axis of the rotary shaft 121 by means of the rotary shaft 121, by providing such an obstruction plate 450a at the bottom 109 of the culture tank 110, the obstruction plate 450a can prevent the culture medium 116 from rotating freely within the culture tank 110, thus preventing the formation of a large vortex (swirling current). As a result, a large amount of culture medium 116 can be ejected from the ejection portions 125, and the efficiency of mixing and stirring the culture medium 116 in the culture tank 110 can be increased.

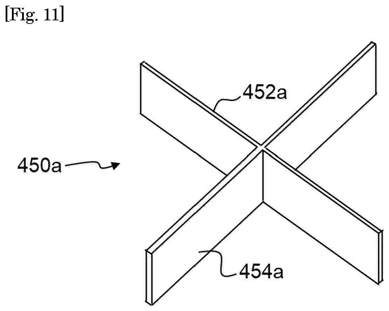

FIG. 11 is a diagram schematically showing an example of an obstruction plate that can be incorporated into the photosynthetic organism culture device of the present invention.

As shown in FIG. 11, the obstruction plate 450a is configured to have a structure in which two plates 452a and 454a intersect each other at an intersection angle of approximately 90°. The obstruction plate 450a is also designed to be large enough to be placed on the bottom of the culture tank of the photosynthetic organism culture device. The materials that form the obstruction plate 450a is not particularly limited, and any material having sufficient durability against a treatment liquid and a predetermined strength may be employed.

Referring again to FIG. 9, according to the culture device 400, by rotating the rotary shaft 121, the formation of vortexes at the liquid surface 128 of the culture medium 116 is suppressed through the obstruction plate 450a, and the liquid flow members 423 of the liquid dispersion portion 420 scoop up the culture medium 116 from the suction ports 424 together with the photosynthetic organisms 117. The scooped up culture medium is moved through the cylindrical paths 426 to the ejection ports 425 by centrifugal force caused by the rotation of the rotary shaft 121, and then ejected from the ejection ports 425 into the culture tank 110, specifically, to portions above the liquid surface 128 of the culture medium 116 in the culture tank 110, together with the photosynthetic organisms 117. When the rotation of the rotary shaft 121 continues and the inside of the cylindrical flow paths 126 is completely filled with the culture medium, the culture medium can move in a "siphon-like manner" among the suction portions 424, the cylindrical flow paths 426, and the ejection portions 425. At this time, even when the rotational speed of the rotary shaft 121 is reduced, the "siphon-like" phenomenon continues due to hysteresis. This allows the culture medium 116 to collide with the inner wall 111 of the culture tank 110 and the liquid surface 128 and to move upward from the bottom 109 of the culture tank 110 together with the photosynthetic organisms 117, thereby facilitating mixing up (e.g., stirring or circulation in the vertical direction) of the culture medium 116 in the height direction of the culture tank 110, by means of suppressed rotation of the rotary shaft 121 (with less power). Meanwhile, light from the outside enters the inside of the culture tank 110 through the light-transmitting portions 132, and thus photosynthesis of the photosynthetic organisms 117 in the culture tank 110 can be facilitated. As a result, the photosynthetic organisms 117 in the culture device 400 can be effectively cultured.

Although the structure shown in FIG. 11 is described above as an example of the obstruction plate, the obstruction plate that can be attached to the culture device of the present invention is not limited to this. The obstruction plate may be, for example, one or more thin and elongate plate-shaped body placed on the inner wall 111 of the culture tank 110 shown in FIG. 9 at a height such that a portion or the entirety of the obstruction plate is immersed in the culture medium 116.

The culture device of the present invention can effectively circulate and stir a culture medium by means of the liquid dispersion portion provided inside the culture tank, and light from the outside is allowed to effectively enter the inside of the culture tank through the light-transmitting portions provided in the culture tank, thereby facilitating the photosynthesis of the photosynthetic organisms contained in the culture medium.

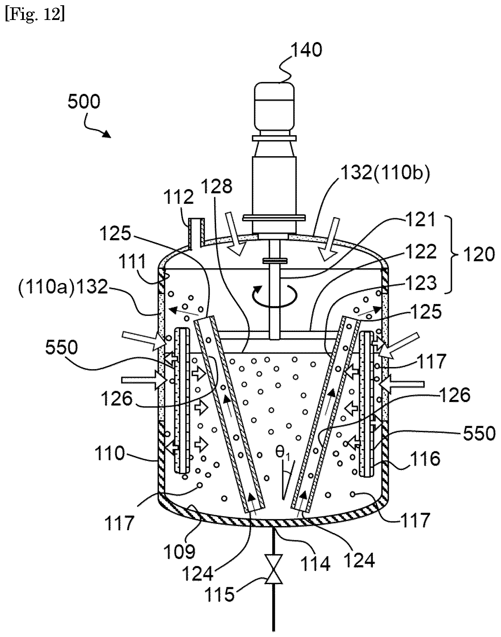

FIG. 12 is a schematic view showing yet another example of the photosynthetic organism culture device of the present invention. A culture device 600 shown in FIG. 12 has a configuration similar to that of the culture tank device 100 shown in FIG. 1, except that internal light sources 550 are provided inside the culture tank 110.

The internal light sources 550 shown in FIG. 12 are light sources (e.g., an LED) that have been pre-treated to be waterproof, and disposed above the liquid surface 128 of the culture medium 116 that is allowed to stand. Lower ends of the internal light sources 550 may be positioned to be immersed in the culture medium 116 as shown in FIG. 12. On the other hand, upper ends of the internal light sources 550 are positioned in the vicinities of the ejection ports 125 of the liquid flow members 123, or preferably near the lower side of the ejection ports 125 of the liquid flow members 123. This is because the culture medium 116 ejected from the ejection ports 125 can readily flow down not only along the inner wall 111 of the culture tank 110 but also along the surfaces of the internal light sources 550.

The internal light sources 550 are electrically connected to the outside of the culture tank 110 by means of wires (not shown). This allows the photosynthetic organisms 117 contained in the culture medium 116 to be irradiated with light applied from the inside of the culture tank 110 in addition to light entering through the light-transmitting portions 117. For example, when the photosynthetic organism culture device 600 of the present invention is placed outdoors, even when light can no longer enter through the light-transmitting portions 117 due to sunset, the culture medium 116 can be irradiated with light inside the culture tank 110 without placing other light-emitting means (e.g., fluorescent lamps or LEDs) outside the culture tank 110.

The internal light sources 550 also preferably have a circular cylindrical shape as shown in FIG. 12, and have a structure in which the inside of the circular cylinder can also emit light. This is because the light emitting area of the internal light sources 550 is enlarged, and the culture medium 116 ejected from the ejection ports 125 of the liquid flow members 123 flows down along the outer surfaces and the inner surfaces of the internal light sources 550, thereby further facilitating photosynthesis of the photosynthetic organisms 117 contained in the culture medium 116.

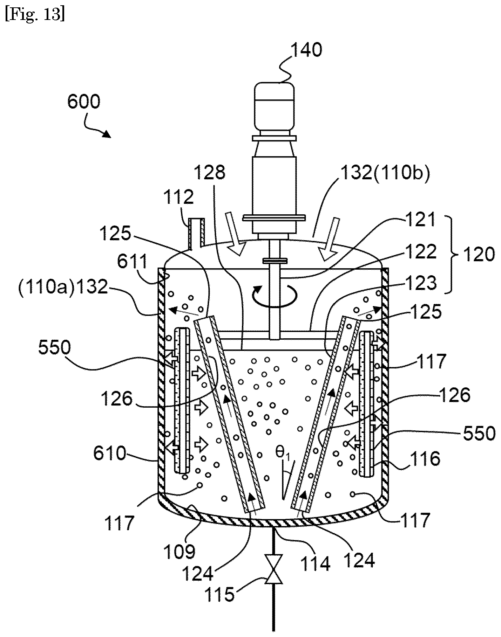

FIG. 13 is a schematic view showing yet another example of the photosynthetic organism culture device of the present invention. A culture device 700 shown in FIG. 13 is similar to the culture device 600 shown in FIG. 12, except that the culture device 700 is configured such that the culture tank 610 does not have a light-transmitting portion and internal light sources 550 are provided inside the culture tank 610.

The culture device 700 shown in FIG. 13 can be used even in an environment where it is difficult to perform light irradiation from the outside of the culture tank 610, for example. In the culture device 700 shown in FIG. 13, the photosynthesis of the photosynthetic organisms 117 in the culture medium 116 can be facilitated only by turning ON/OFF the internal light sources 550.

### Method for Culturing Photosynthetic Organism

A culture method described below can be used to culture the photosynthetic organisms using the above culture device, for example.

First, in the culture method of the present invention, the culture medium containing photosynthetic organisms is stirred under light irradiation, by rotating the rotary shaft in the device for culturing photosynthetic organisms.

When stirring the culture medium, carbon dioxide gas or the like necessary for culturing the photosynthetic organisms may be supplied to the culture medium continuously or discontinuously. Also, the culture tank may be heated to a predetermined temperature in order to more efficiently culture the photosynthetic organisms.

Note that light is emitted toward the light-transmitting portions provided in the culture tank in the culture device. The type of light is not particularly limited, and may be, for example, sunlight (natural light) or artificial light such as a halogen lamp, a xenon lamp, or an LED. The light to be emitted may be obtained by, for example, cutting, using a bandpass filter or the like, short wavelengths that may inhibit the growth or proliferation of photosynthetic organisms.

The above stirring is performed continuously or intermittently during light irradiation. For example, when sunlight is used, the stirring may be stopped at night because sunlight is not available, or the stirring can be continued or extended by irradiating the culture tank with artificial light at night.

Photosynthetic organisms can be cultured in the culture device in this manner.

### Examples

Hereinafter, the present invention will be described in detail using examples. However, the present invention is not limited to these examples.

### Example 1: Fabrication of Photosynthetic Organism Culture Device (R1)

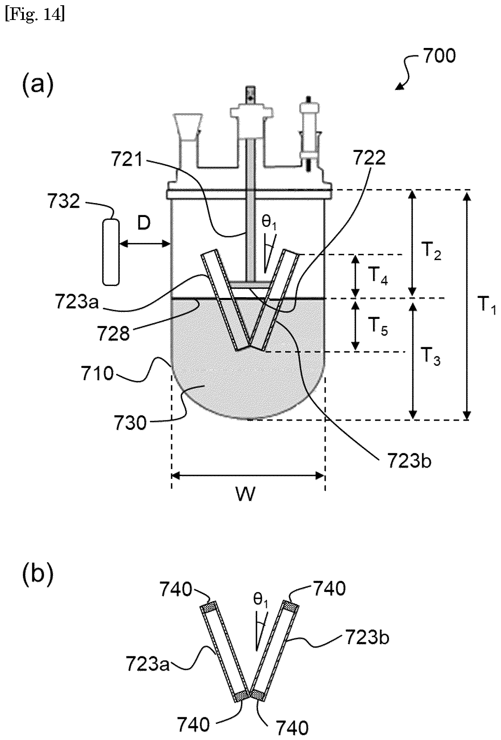

A photosynthetic organism culture device (R1) 700 shown in FIG. 14(a) was fabricated as follows. Specifically, liquid flow members 723a and 723b that were respectively composed of two circular cylindrical stainless steel pipes (with an inner diameter of 10 mm and a length of 86 mm) were fixed in a V-shape form with an attachment 722 so as to be arranged facing each other and inclined at 20° (θ₁) with respect to the axial direction of a rotary shaft 721 in a round-bottomed and transparent glass culture tank 510 with an inner diameter (W) of 130 mm and a depth (T₁) of 220 mm, thereby disposing the liquid dispersion portion.

### Comparative Example 1: Fabrication of Photosynthetic Organism Culture Device (R2)

A photosynthetic organism culture device (R2) was fabricated in the same way to that of Example 1, except that a liquid dispersion portion (FIG. 14(b)), in which lower ends and upper ends of the liquid flow members 723a and 723b were sealed with silicone resin plugs 740, was disposed instead of the liquid dispersion portion in the photosynthetic organism culture device (R1) 700 shown in FIG. 14(a).

### Example 2: Culturing (1) of Microalgae (Euglena) using Photosynthetic Organism Culture Device (R1)

A stock culture solution was prepared which contained *Euglena* (*Euglena gracilis* NIES-48) (available from the Microbial Culture Collection at the National Institute for Environmental Studies) cultured in HUT medium at 25°C under the following culture conditions: a light/dark cycle of 10 L (LED ON) : 14 D (LED OFF), a light intensity of approximately 14.7 µmol/(m²•sec), and a subculture cycle of approximately 1 month. Then, 10 mL of the stock culture solution was inoculated into 100 mL of the HUT medium not containing agar in an Erlenmeyer flask, and static culturing was carried out for 4 days under the same culture conditions as above to obtain a preculture medium.

Thereafter, 100 mL of the preculture medium was introduced into a separable flask containing 1000 mL of a modified KH medium (pH 3.5) in a laminar flow cabinet, and main culturing was carried out. During main culturing, the flask was kept at approximately 28°C in a water bath.

Then, the main culture medium was placed into a culture tank 710 of the photosynthetic organism culture device (R1) 700 fabricated in Example 1. Here, a distance T₂ from a liquid surface 728 that was allowed to stand and shown in FIG. 14(a) to the upper end of the culture tank 710 was 115 mm, a distance T₃ from the liquid surface 728 to the bottom of the culture tank 710 was 105 mm, a distance T₄ from the liquid surface 728 to an upper end of the liquid flow member 723b was 35 mm, and a distance T₅ from the liquid surface 728 to a lower end of the liquid flow member 723b was 49 mm.

Next, a fluorescent lamp 532 with a vertical length of 50 mm was positioned with a distance D of 10 mm between the fluorescent lamp and the culture tank 710 to emit light toward a gap portion of the culture tank 710 (a portion of the culture tank 710 in which a culture medium 730 was not placed), such that the light intensity at the shortest irradiation distance was approximately 600 µmol/(m²•sec). Note that light irradiation was performed using the fluorescent lamp in a dark place.

The fluorescent lamp 732 was turned on in this state, and the rotary shaft 721 was rotated at a rotational speed of 300 rpm at room temperature, thereby culturing *Euglena* in the photosynthetic organism culture device (R1). During culturing, the culture medium in the culture tank 710 was periodically sampled, and the turbidity (OD) at 600 nm of the obtained culture medium was measured using an absorption photometer (U-2800Amanufactured by Hitachi High-Tech Corporation).

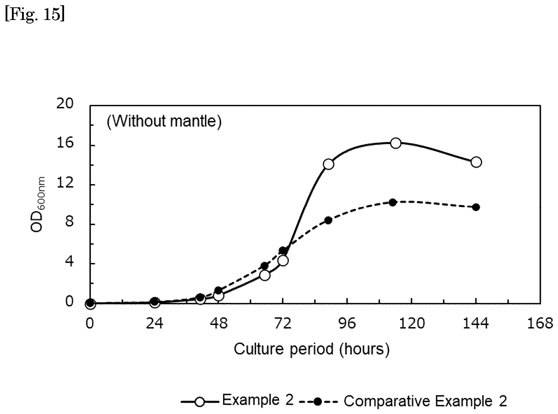

Culturing of *Euglena* in the photosynthetic organism culture device (R1) was continued for 6 days (144 hours). The results of measuring the turbidity of the culture medium obtained through sampling during the culture period are shown in FIG. 15.

### Comparative Example 2: Culturing (1) of Microalgae (Euglena) using Photosynthetic Organism Culture Device (R2)

*Euglena* was cultured for 6 days (144 hours) in the same manner as in Example 2, except that the photosynthetic organism culture device (R2) (using a liquid dispersion portion in which the lower ends and the upper ends of the liquid flow members 723a and 723b were sealed with silicone resin 740; FIG. 14(b)) fabricated in Comparative Example 1 was used instead of the photosynthetic organism culture device (R1) used in Example 2, and the turbidity of the culture medium obtained through sampling during the culture period was measured. FIG. 15 shows the results.

### Example 3: Culturing (2) of Microalgae (Euglena) using Photosynthetic Organism Culture Device (R1) equipped with Mantle

In the photosynthetic organism culture device (R1) shown in FIG. 14(a), in order to eliminate influence of light emitted from the fluorescent lamp 732 directly onto the culture medium 730 in the culture tank 710, the portion of the photosynthetic organism culture device (R1) that contained the culture medium 730 (below the liquid surface 728 that was allowed to stand) was shaded with an aluminum foil (mantle) (accordingly, only the gap portion of the culture tank 710 was irradiated with light from the fluorescent lamp 732).

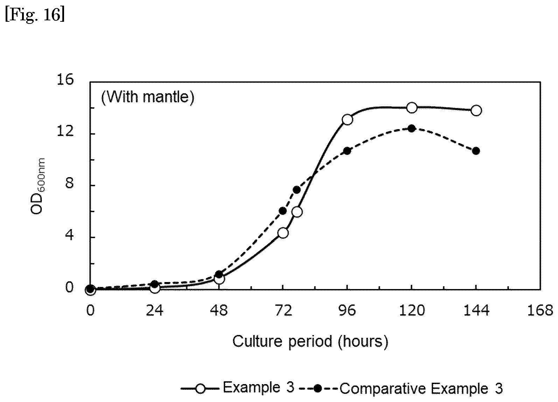

*Euglena* was cultured for 6 days (144 hours) in the same manner as in Example 2, except that the photosynthetic organism culture device (R1) equipped with such a mantle was used, and the turbidity of the culture medium obtained through sampling during the culture period was measured. FIG. 16 shows the results.

### Comparative Example 3: Culturing (2) of Microalgae (Euglena) using Photosynthetic Organism Culture Device (R2) equipped with Mantle

The photosynthetic organism culture device (R2) (using the liquid dispersion portion in which the lower ends and the upper ends of the liquid flow members 723a and 723b were sealed with silicone resin 740; FIG. 14(b)) fabricated in Comparative Example 1 was used, and a portion of the photosynthetic organism culture device (R2) that contained the culture medium (below the liquid surface that was allowed to stand) was shaded with an aluminum foil (mantle) in the same manner as in Example 3. *Euglena* was cultured for 6 days (144 hours) in the same manner as in Example 2, except that the photosynthetic organism culture device (R2) equipped with such a mantle was used, and the turbidity of the culture medium obtained through sampling during the culture period was measured. FIG. 16 shows the results.

As shown in FIG. 15, when the photosynthetic organism culture device (R1) fabricated in Example 1 was used, 72 hours after starting culturing, the density of *Euglena* in the culture tank increased rapidly, and photosynthesis of *Euglena* was more effectively facilitated than in the photosynthetic organism culture device (R2) fabricated in Comparative Example 1. On the other hand, as shown in FIG. 16, even when the portion that contained the culture medium was shaded with the mantle and only the upper side of the liquid dispersion portion was irradiated with light, the density of *Euglena* in the culture tank in the photosynthetic organism culture device (R1) equipped with the mantle more rapidly increased about 72 hours after starting the culturing than in the photosynthetic organism culture device (R2) equipped with the mantle.

### Example 4: Culturing (3) of Microalgae (Chlorella) using Photosynthetic Organism Culture Device (R1)

*Chlorella* (*Chlorella vulgaris* NIES-2170) (available from the Microbial Culture Collection at the National Institute for Environmental Studies), which was cultured in agar-free C medium at 25°C under the following culture conditions: a light/dark cycle of 10 L (LED ON) : 14 D (LED OFF) and a light intensity of approximately 14.7 µmol/(m²•sec), was subcultured and transferred to an Erlenmeyer flask containing 100 mL of basal medium (pH 6.0; Table 1) containing glucose, and subjected to static culturing for 4 days under the same culture conditions as above to obtain a preculture medium.

**[Table 1] Bassal medium used in Example 4**

| | |
|---|---|
| Glucose | 2% |
| Yeast extract | 0.1% |
| Sodium nitrate | 0.2% |
| K₂HPO₄ | 0.08% |
| MgSO₄▪7H₂O | 0.02% |
| KH₂PO₄ | 0.02% |
| CaCl₂▪2H₂O | 0.005% |
| Ammonium iron citrate | 0.002% |
| Trace metal mixture A5 | 1.0 mL |
| Distilled water | 1000 mL |

The composition of "Trace metal mixture A5" in Table 1 is as follows.

**[Table 2] Composition of trace metal mixture A5**

| | |
|---|---|
| H₃BO₄ | 2.86 g |
| MnCl₂▪4H₂O | 1.81 g |
| ZnSO₄▪7H₂O | 0.22 g |
| CuSO₄▪5H₂O | 0.08 g |
| Na₂MoO₄ | 0.021 g |
| CaCl₂▪6H₂O | 0.01 g |
| EDTA▪2Na | 50 g |
| Distilled water | 1000 mL |

Then, 100 mL of the preculture medium was introduced into a separable flask containing 1000 mL of a main culture medium candidate (pH 6.0; the above-mentioned basal medium) in a laminar flow cabinet, and main culturing was carried out at 25°C (C medium was used for subculturing).

Then, the main culture medium was placed into the culture tank 710 of the photosynthetic organism culture device (R1) 700 fabricated in Example 1. Here, the distance T₂ from the liquid surface 728 that was allowed to stand and shown in FIG. 14(a) to the upper end of the culture tank 710 was 120 mm, the distance T₃ from the liquid surface 728 to the bottom of the culture tank 710 was 95 mm, the distance T₄ from the liquid surface 728 to the upper end of the liquid flow member 723b was 35 mm, and the distance T₅ from the liquid surface 728 to the lower end of the liquid flow member 723b was 49 mm.

Next, the fluorescent lamp 732 with a vertical length of 50 mm was positioned with a distance D of 10 mm between the fluorescent lamp and the culture tank 710 to emit light toward the gap portion of the culture tank 710 (the portion of the culture tank 710 where no culture medium 730 was placed), such that the light intensity at the shortest irradiation distance was approximately 600 µmol/(m²•sec). Note that light irradiation was performed using the fluorescent lamp in a dark place.

The fluorescent lamp 732 was turned on in this state, and the rotary shaft 721 was rotated at a rotational speed of 300 rpm at room temperature, thereby culturing *Chlorella* in the photosynthetic organism culture device (R1). During culturing, the culture medium in the culture tank 710 was periodically sampled, and the turbidity (OD) at 600 nm of the obtained culture medium was measured using an absorption photometer (U-2800Amanufactured by Hitachi High-Tech Corporation).

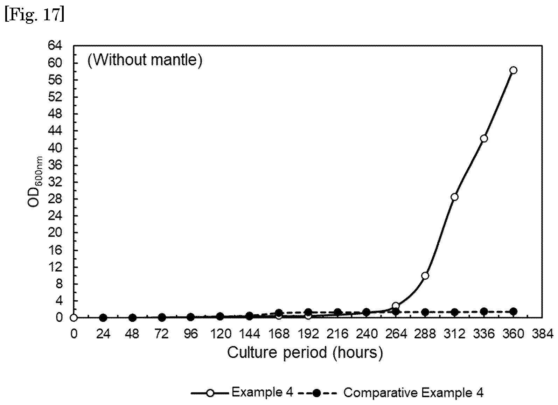

Culturing of *Chlorella* in the photosynthetic organism culture device (R1) was continued for 15 days (360 hours). The results of measuring the turbidity of the culture medium obtained through sampling during the culture period are shown in FIG. 17.

### Comparative Example 4: Culturing (3) of Microalgae (Chlorella) using Photosynthetic Organism Culture Device (R2)

*Chlorella* was cultured for 15 days (360 hours) in the same manner as in Example 4, except that the photosynthetic organism culture device (R2) (using a liquid dispersion portion in which the lower ends and the upper ends of the liquid flow members 723a and 723b were sealed with the silicone resin 740; FIG. 14(b)) fabricated in Comparative Example 1 was used instead of the photosynthetic organism culture device (R1) of Example 1 used in Example 4, and the turbidity of the culture medium obtained through sampling during the culture period was measured. FIG. 17 shows the results.

As shown in FIG. 17, up until 264 hours after starting culturing, no significant change was observed in the turbidity of the culture medium, despite using the photosynthetic organism culture device (R1) used in Example 2 or the photosynthetic organism culture device (R2) fabricated in Comparative Example 1. *Chlorella* was photoinhibited in the early stages of culturing under the light irradiation conditions used in this study, suggesting that there is room for optimization of the culture conditions.

On the other hand, as shown in FIG. 17, 264 hours after starting culturing, the turbidity of the culture medium in Example 4 suddenly increased compared to that in Comparative Example 4, indicating that *Chlorella* cells rapidly proliferated in the culture medium. A portion of the culture medium in Example 4 was actually collected and the collected culture medium was observed using a phase contrast microscope (a magnification of 400), and it was confirmed that proliferated *Chlorella* cells were spread substantially uniformly and in large quantities within the field of view of the microscope. In contrast, a portion of the culture medium in Comparative Example 4 was observed in the same manner using the phase contrast microscope (a magnification of 400), and it was confirmed that *Chlorella* cells having distorted cell shapes were spread out at intervals in the field of view of the microscope.

Furthermore, 360 hours after starting culturing, the number (concentration) of cells contained in each culture medium of Example 4 and Comparative Example 4 was measured using a cell counter (Cell Counter model R1 manufactured by Olympus Corporation). For this measurement, the culture medium of Example 4 was diluted into 100 times the medium, with the main culture medium candidate in advance and then used.

As a result of measurement, it was confirmed that the culture medium of Example 4 contained *Chlorella* cells with an order of approximately 10⁸ cells/mL (value converted to the culture medium before dilution), and the culture medium of Comparative Example 4 contained *Chlorella* cells with an order of approximately 10⁶ cells/mL.

This indicates that the photosynthetic organism culture device (R1) of Example 1 having the liquid dispersion members with open ends can more effectively facilitate photosynthesis in photosynthetic organisms such as *Euglena* and *Chlorella,* compared to the photosynthetic organism culture device (R2) of Comparative Example 1, in which both ends of each liquid flow member were closed.

### Industrial Applicability

According to the present invention, it is possible to efficiently culture industrially useful photosynthetic organisms. The photosynthetic organisms obtained according to the present invention are useful as, for example, a raw material for foods or cosmetics. By culturing the photosynthetic organisms, the photosynthetic organisms can also be used to produce useful substances such as biofuels.

### Reference Signs List

100, 200, 300, 400, 500, 600, 700 Photosynthetic organism culture device
109 Bottom
110, 510, 610, 710 Culture tank
110a Wall surface portion
110b Lid portion
111, 611 Inner wall
112 Culture medium inlet
114 Culture medium outlet
115 Valve
116 Culture medium
117 Photosynthetic organism
120, 220, 320, 420 Liquid dispersion portion
121, 321, 721 Rotary shaft
122,722 Attachment
123, 223, 423, 723a, 723b Liquid flow member
124, 224, 424 Suction portion
125, 225, 425 Ejection portion
126, 226, 426 Flow path
128, 528 Liquid surface
132 Light-transmitting portion
134 Support
136 Upper frame
138 Lower frame
140, 340 Motor
450a Obstruction plate
550 Internal light source
732 Fluorescent lamp
740 Silicone resin plug

## Claims

1. A device for culturing a photosynthetic organism, comprising:
a culture tank for accommodating a culture medium containing the photosynthetic organism; and
a liquid dispersion portion provided inside the culture tank,
wherein the liquid dispersion portion includes one rotary shaft disposed along a vertical direction and at least one liquid flow member attached to the rotary shaft,
the liquid flow member includes an ejection portion positioned above a liquid surface of the culture medium, a suction portion positioned below the liquid surface of the culture medium, and a flow path extending between the ejection portion and the suction portion and allowing the culture medium to flow therethrough, and
at least a part of the culture tank includes a light-transmitting portion that allows transmission of light from an outside into an inside of the culture tank.

2. The device for culturing a photosynthetic organism according to claim 1, wherein the light-transmitting portion is provided in at least one region selected from the group consisting of a wall surface portion and a lid portion of the culture tank.

3. The device for culturing a photosynthetic organism according to claim 2, wherein the light-transmitting portion is provided on a wall surface portion of the culture tank, the culture medium is ejected from the ejection portion of the liquid flow member by rotating the rotary shaft in the liquid dispersion portion, and the culture medium flows down on an inner side along the wall surface portion of the culture tank to form a thin film.

4. The device for culturing a photosynthetic organism according to claim 1, wherein the liquid flow member is inclined such that the suction portion is closer to the rotary shaft than the ejection portion is.

5. The device for culturing a photosynthetic organism according to claim 1, wherein the liquid dispersion portion includes a plurality of the liquid flow members around an axis of the rotary shaft.

6. The device for culturing a photosynthetic organism according to claim 1, wherein the liquid flow member has a cylindrical shape with open ends.

7. The device for culturing a photosynthetic organism according to claim 1, wherein the liquid flow member has a gutter-like shape.

8. A device for culturing a photosynthetic organism, comprising:
a culture tank for accommodating a culture medium containing a photosynthetic organism; and
a liquid dispersion portion provided inside the culture tank,
wherein the liquid dispersion portion includes one rotary shaft disposed along a vertical direction and at least one liquid flow member attached to the rotary shaft,
the liquid flow member includes an ejection portion positioned above a liquid surface of the culture medium, a suction portion positioned below the liquid surface of the culture medium, and a flow path extending between the ejection portion and the suction portion and allowing the culture medium to flow therethrough, and
the device includes an internal light source disposed inside the culture tank.

9. A method for culturing a photosynthetic organism, comprising:
stirring a culture medium containing the photosynthetic organism under light irradiation, by rotating a rotary shaft in the device for culturing a photosynthetic organism according any one of claims 1 to 8.

10. The method according to claim 9, wherein the light is emitted toward the light-transmitting portion provided in the culture tank in the device for culturing a photosynthetic organism.

11. The method according to claim 9, wherein the photosynthetic organism is at least one organism selected from the group consisting of photosynthetic bacteria, blue-green algae, microalgae, and plant cells.
